# EUROPÄISCHE PATENTSCHRIFT

(11) **EP 2 883 568 B1**
(45) Veröffentlichungstag und Bekanntmachung des Hinweises auf die Patenterteilung: **17.03.2021**
(21) Anmeldenummer: 13196634.3
(22) Anmeldetag: 11.12.2013
(51) Int. Cl.: A61N 5/10

(54) **System zum Ermitteln der Position von Objekten in einem Bestrahlungsraum für eine Strahlentherapie**
System for determining the position of objects in an irradiation room for radiation therapy
Système de détermination de la position d'objets dans une chambre d'irradiation pour radiothérapie

(43) Veröffentlichungstag der Anmeldung: 17.06.2015
(73) Patentinhaber: Hofmann, Karsten, 21337 Lüneburg (DE)
(72) Erfinder: Hofmann, Karsten, 21337 Lüneburg (DE); Kindlein, Johann, 21365 Adendorf (DE)
(74) Vertreter: Hauck Patentanwaltspartnerschaft mbB

(56) Entgegenhaltungen:
- EP-A1- 0 687 443
- WO-A1-2009/088407
- WO-A1-2011/026601
- WO-A1-2011/071442
- WO-A1-2012/118228
- US-A1- 2006 215 813

## Beschreibung

Die Erfindung betrifft ein System zum Ermitteln der Position von Objekten in einem Bestrahlungsraum für eine Strahlentherapie. In derartigen Bestrahlungsräumen findet eine Strahlentherapie von Patienten zur Krebsbehandlung statt. Dabei werden mittels der Bestrahlungsgeräte Tumore mit ionisierender Strahlung bestrahlt. Entscheidend ist die korrekte Positionierung des Patienten, so dass die ionisierende Strahlung den Tumor optimal trifft. In einem von dem Bestrahlungsraum getrennten CT-Raum wird anhand von CT-Aufnahmen das zu bestrahlende Gebiet lokalisiert und es werden Markierungen auf den Patientenkörper aufgebracht, anhand derer der Patient nachfolgend in dem Bestrahlungsraum positioniert wird. Dazu sind in dem Bestrahlungsraum sogenannte Raumlaser angeordnet. Raumlaser sind in dem Bestrahlungsraum fest an der Decke oder der Wand angeordnete Laserprojektoren, die einen oder zwei Lichtfächer erzeugen. In der Regel sind mindestens drei derartige Raumlaser installiert, die auf das Isozentrum des Bestrahlungsgeräts ausgerichtet sind. Anhand der auf dem Patientenkörper aufgebrachten Markierungen und mittels der Raumlaser wird der Patient durch geeignetes Verfahren eines Patiententisches für die Bestrahlung ausgerichtet. Insbesondere werden dabei die auf dem Patientenkörper aufgebrachten Markierungen mit den auf das Isozentrum des Bestrahlungsgeräts ausgerichteten Laserkreuzen in Überdeckung gebracht. Zum Zwecke der Positionierung wird bei dem heutigen Stand der Technik nur ein kleiner Bereich der jeweils projizierten Laserlinien um das Laserkreuz verwendet. Aufgrund der Aufweitung der Lichtfächer werden von den Raumlasern üblicherweise neben dem Patienten auch andere Objekte im Bestrahlungsraum beleuchtet.

Aus DE 103 42 202 A1 ist eine Vorrichtung zur Lageüberwachung eines Patienten bei einer Bestrahlung bekannt, bei dem zwei oder mehr Abstandsmessgeräte den Abstand zu jeweils einem Punkt auf der Haut des Patienten messen. Eine Auswerteeinrichtung bestimmt aus den mindestens zwei Abstandswerten, ob sich die Position des Patienten gegenüber einer Ausgangsposition geändert hat. Für die Abstandsmessung kann die sogenannte Off-Axis-Triangulation eingesetzt werden. Dabei werden allerdings nur wenige einzelne Punkte auf der Haut des Patienten erfasst, so dass eine genaue und umfassende Überwachung der Position des Patientenkörpers nicht in zufriedenstellender Weise möglich ist. Außerdem ist die bekannte Vorrichtung konstruktiv aufwendig, da die Abstandsmessgeräte zusätzlich in dem Bestrahlungsraum untergebracht werden müssen. Darüber hinaus ist aus DE 297 24 767 U1 eine Vorrichtung zum Erfassen der Position eines sich in einem Bestrahlungsraum befindlichen Objekts bekannt. Dabei soll eine Kollision von Komponenten der medizinischen Einrichtung, beispielsweise eines Strahlensenders, mit anderen sich im Raum befindlichen Objekten verhindert werden. Eingesetzt werden kann eine triangulierende 3D-Technik. Auch diese bekannte Vorrichtung ist konstruktiv aufwendig, da die für die Messung eingesetzten Lichtsender und Kameras zusätzlich in dem Bestrahlungsraum untergebracht werden müssen.

Aus WO 2011/071442 A1 sind ein System und ein Verfahren zur Positionierung eines Patienten bekannt. Ein Musterprojektor projiziert ein Muster auf einen Patienten, das über Kameras detektiert wird. Aus dem detektierten Signal wird eine Oberflächendarstellung des Patienten erstellt und mit einer gespeicherten Referenzdarstellung verglichen. Eine Abweichung der beiden Darstellungen wird ermittelt und mittels eines weiteren Projektors auf den Bauch des Patienten projiziert.

Aus WO 2012/118228 A1 und WO 2009/088407 A1 sind Methoden zur Berücksichtigung der Atmung und der damit einhergehenden Bewegung eines zu bestrahlenden Tumors eines Patienten während der Bestrahlungsbehandlung bekannt.

EP 0 687 443 A1 beschreibt eine Vorrichtung zur Positionierung und Markierung eines Patienten mit verfahrbaren Linienlasern im Anschluss an eine CT-Aufnahme. WO 2011/026601 A1 beschreibt die Darstellung einer geometrischen Figur auf der Körperoberfläche eines Patienten in einem Behandlungsraum, welche mit starren oder beweglichen Lasern auf die Oberfläche des Patienten projiziert und mit einer Kamera aufgenommen wird.

Der eingangs erläuterte Vorgang der Patientenpositionierung beruht auf einer subjektiven Bewertung durch den jeweiligen Anwender. Es ist eine unumstrittene Tatsache, dass die auf der Haut des Patienten markierten Punkte zur Positionierung des Patienten für die heutige moderne Strahlentherapie mit hohen Dosen pro Bestrahlungsfraktion und kleinen Bestrahlungsfeldern bei hohen Feldgradienten nicht mehr den Genauigkeitsanforderungen entsprechen. Neue bildgebende Verfahren wie CB-CT, Ultraschall oder MRT halten Einzug in den Bestrahlungsraum und werden heute schon in diesen integriert. Multimodale Bildregistrierungsmethoden mit rigiden (RIR) oder elastischen (deformierbaren DIR) Algorithmen und Bildpositionierungsmethoden sind aus der heutigen Strahlentherapie nicht mehr wegzudenken. Trotzdem kommt auch die heutige Strahlentherapie an einer möglichst genauen Anfangspositionierung des Patienten mit Laserlinien nicht vorbei. Bildpositionierungsalgorithmen verwenden spezielle Optimierungsverfahren, um die vor der Bestrahlung erstellten 3D-Aufnahmen mit einer 3D-CT-Referenzposition zu vergleichen. Ist aufgrund einer fehlerhaften Patientenpositionierung die Patientenanfangsposition nicht ausreichend nah an der Referenzposition, können diese Optimierungsverfahren falsche Ergebnisse liefern. Hierdurch kann es zu falschen Positionierungsangaben (Verschiebungsvektoren) und somit zu einer nicht planungsgemäßen Patientenbestrahlung kommen.

Das Aufnehmen von CB-CT-Bildern vor jeder Bestrahlungsfraktion ist zeitaufwendig und mit jeder Aufnahme steigt die Strahlenbelastung der gesunden Organe des Patienten, was zu einer nachträglichen strahleninduzierten Krebserkrankung führen kann. Besonderes Augenmerk muss in dieser Hinsicht auf die Behandlung von Kindern und jungen erwachsenen Patienten gerichtet sein.

Es besteht daher ein weiter wachsender Bedarf, die Patientenpositionierung für die Bestrahlung und während der Bestrahlung mit höchster Genauigkeit und ohne zusätzliche Strahlenbelastung vornehmen zu können. Darüber hinaus bestehen bei den oben erläuterten modernen Bestrahlungsgeräten mit hohen Dosisleistungen, steilem Feldgradienten und kurzen Behandlungszeiten immer höhere Anforderungen an die Genauigkeit der zur Bestrahlung eingesetzten Geräte. Dies gilt insbesondere bei der intensitätsmodulierten Strahlentherapie (IMRT, VMAT). Bekanntlich dreht sich der Kopf eines zur Bestrahlung eingesetzten Linearbeschleunigers (Gantry) während der Bestrahlung um den Patienten. Beispielsweise bei der VMAT-Technologie findet die Modulation der Strahlungsintensität mit einer Veränderung der Drehgeschwindigkeit der Gantry auf bestimmten Kreispositionen und durch die unterschiedlichen Öffnungen der Multi-Leaf Kollimatoren (MLC) statt. Treten beispielsweise im Zuge der Kreisbewegung der Gantry Positionsabweichungen auf, wie sie beispielsweise durch das hohe Gewicht der Gantry verursacht werden können, wirkt sich dies unzulässig auf das Bestrahlungsergebnis aus. Außerdem hat sich herausgestellt, dass auch die Positionsgenauigkeit eines den Patienten lagernden Patiententisches eine große Rolle spielt. Bereits kleinste Abweichungen, wie sie sich beispielsweise aufgrund unterschiedlichen Gewichts von Patienten ergeben, wirken sich bei den heutigen hochpräzisen Bestrahlungsverfahren ungünstig auf die Qualität der Bestrahlungsfraktion aus.

Ausgehend von dem erläuterten Stand der Technik liegt der Erfindung die Aufgabe zugrunde, ein System und ein Verfahren der eingangs genannten Art bereitzustellen, mit denen in konstruktiv einfacher aber dennoch präziser Weise die Position von Objekten in einem Bestrahlungsraum für eine Strahlentherapie bestimmt werden kann. Außerdem sollen unzulässige Positionsabweichungen erkannt werden und es sollen nachfolgende Bestrahlungsvorgänge optimiert werden.

Die Erfindung löst die Aufgabe durch den Gegenstand des unabhängigen Anspruchs 1. Vorteilhafte Ausgestaltungen finden sich in den abhängigen Ansprüchen, der Beschreibung und den Figuren.

Das erfindungsgemäße System kann insbesondere das Bestrahlungsgerät und den Patiententisch umfassen. In dem Bestrahlungsraum befindet sich in an sich bekannter Weise ein Bestrahlungsgerät für eine Krebstherapie eines Patienten. Bei dem Bestrahlungsgerät handelt es sich insbesondere um einen Linearbeschleuniger (LINAC), dessen Kopf (Gantry) sich während einer Bestrahlung um einen den Patienten tragenden Patiententisch dreht. Erfindungsgemäß ist vorgesehen, die im Bestrahlungsraum fest angeordneten eingangs erläuterten Raumlaser, die zur Positionierung eines Patienten für die Bestrahlung genutzt werden, für weitere Funktionen einzusetzen, nämlich für die Positionsbestimmung eines Patienten und gegebenenfalls weiterer Objekte im Bestrahlungsraum. Hierzu wird das an sich bekannte Verfahren der Laser-Triangulation genutzt. Dazu wird mindestens eine Laserlinie, vorzugsweise mehrere Laserlinien, zumindest auf den Patienten projiziert und mindestens eine Laserlinie, vorzugsweise sämtliche Laserlinien, werden von einer oder mehreren hochauflösenden Kameras aufgenommen. Durch die Nutzung der für die Patientenpositionierung eingesetzten und ohnehin vorhandenen Raumlaser wird das erfindungsgemäße System konstruktiv vereinfacht. Anders als im Stand der Technik werden darüber hinaus nicht nur wenige einzelne Punkte auf der Oberfläche des Patienten hinsichtlich ihres Abstandes zur Gantry gemessen, sondern es werden die 3D-Koordinaten entlang des jeweils ausgewerteten Abschnitts der Laserlinien durch das Messverfahren der Triangulation ermittelt. Die Laserlinien können zum Beispiel durch Zylinderlinsen erzeugt werden. Die Raumlaser können grundsätzlich in beliebigen Wellenlängenbereichen Laserlicht aussenden, vorzugsweise jedoch im sichtbaren Bereich. Es können eine oder mehrere Kameras in dem Bestrahlungsraum vorgesehen sein, so dass im Maximalfall jede von einem Raumlaser projizierte Laserlinie durch eine Kamera erfasst wird. Das Sichtfeld der Kameras ist so gewählt, dass wahlweise die gesamten der Kamera zugewandten Laserlinien mittels eines Weitwinkelobjektivs erfasst werden, oder nur bestimmte interessierende Abschnitte bei Verwendung eines Objektivs mit entsprechend eingeschränktem Sichtfeld. Bei Verwendung mehrerer Kameras ist auch eine Kombination aus beidem möglich.

Die Auswerte- und Steuereinrichtung umfasst einen Computer mit einer geeigneten Software, mit der die Auswertung der Kamerabilder möglich ist. Auch können damit die Raumlaser bzw. die Kameras angesteuert werden und Messdaten der Kameras ausgelesen werden. Mittels der Software können daraus die 3D-Koordinaten der insbesondere auf dem Patienten als eines der Objekte in dem Bestrahlungsraum projizierten Laserlinien ermittelt werden. Dabei kann die Software auch DICOM-RT Informationen importieren. Außerdem kann sie insbesondere ein Datenbanksystem besitzen. Auch ist es möglich, mehrere in dem Bestrahlungsraum befindliche Objekte im Rahmen der Auswertung zu erfassen und deren relative Position zueinander zu bestimmen.

Die Erfindung erlaubt in konstruktiv einfacher und kosteneffektiver Weise die Echtzeit-Positionsüberwachung und Erkennung einer Positionsveränderung unter Nutzung der ohnehin im Bestrahlungsraum vorhandenen Raumlaser. Es sind keine zusätzlichen Laser für die Messung erforderlich. Vielmehr werden die ohnehin vorhandenen Raumlaser für weitere neue Funktionalitäten verwendet, insbesondere zur Überwachung der Patientenposition und gegebenenfalls der Position weiterer Objekte während der Bestrahlung. Es wird dadurch ein multifunktionales, kostengünstiges und zeiteffektives System bereitgestellt. Anhand des Vergleichs der ermittelten Koordinatenpunkte entlang der Laserlinien mit Sollkoordinatenpunkten kann eine unzulässige Abweichung der Patientenlage ermittelt werden und es können geeignete Gegenmaßnahmen ergriffen werden. Die Sollkoordinaten können die zu Beginn der Bestrahlung nach endgültiger Positionierung des Patienten gemessenen und gespeicherten Koordinaten sein. In diesem Fall wird also eine Veränderung der Patientenposition gegenüber der ursprünglich ausgerichteten Position ermittelt. Auch die Patientenpositionierung insbesondere zu Beginn einer Bestrahlungsfraktion wird durch die Erfindung verbessert, da die Positionierung nicht mehr ausschließlich auf (verschieblichen) Hautmarkierungen basiert, sondern auf der Übereinstimmung ganzer Körperkonturprofile.

Weiterhin werden erfindungsgemäß zumindest die während des Bestrahlungsvorgangs ermittelten Koordinatenpunkte insbesondere von Patient und Gantry für eine Dokumentation des Bestrahlungsvorgangs gespeichert. Durch das Speichern der während einer Bestrahlungsfraktion ermittelten Positionsdaten kann eine genaue Dokumentation der bei jeder Bestrahlungsfraktion von dem Patienten erhaltenen Dosis mit genauer Lokalisierung des Einwirkungsbereichs der Strahlung erstellt werden. Auf dieser Grundlage kann die Qualität einer Bestrahlungsfraktion präzise bewertet werden und es können nachfolgende Bestrahlungsfraktionen gegebenenfalls in geeigneter Weise angepasst werden. Insbesondere können Abweichungen der in einer Bestrahlungsfraktion erhaltenen Dosis von einer Solldosis in einer nachfolgenden Bestrahlungsfraktion kompensiert werden.

Dabei kann die Messung der Profilform der im Blickfeld der Kameras liegenden Laserlinien zur Bestimmung der Koordinaten in der jeweiligen Schnittebene vorzugsweise im Koordinatensystem des Bestrahlungsgerätes erfolgen. Die vorhandenen Kamerakoordinatensysteme werden dafür mit Hilfe eines an sich bekannten geeigneten Kalibrierverfahrens in ein gemeinsames Raumkoordinatensystem transformiert . Wie erläutert, wird vorzugsweise der Schnittpunkt dreier jeweils orthogonal zueinander angeordneter Laserebenen als Nullpunkt des Raumkoordinatensystems gewählt. Die erfindungsgemäß bestimmten Schnittpunktkoordinaten können dann in diesem Raumkoordinatensystem bestimmt werden. Wenn beispielsweise drei Laserlinien mittels Kameras erfasst und hinsichtlich der Koordinatenpunkte ausgewertet werden, liefert jede der Laserlinien eine Koordinatenschar. Beispielsweise sollen die von den Raumlasern erzeugten Laserebenen sich im Isozentrum des Bestrahlungsgeräts schneiden, welches beispielsweise die Koordinaten (0,0,0) in dem Raumkoordinatensystem besitzt. Eine erste Laserlinie liefert dann die Koordinatenwerte (x,y,0). Eine zweite Laserlinie liefert die Koordinatenwerte (x,0,z). Eine dritte Laserlinie liefert die Koordinatenwerte (0,y,z). Mit dieser Koordinatenschar kann die Patientenposition eindeutig ermittelt werden.

Da die Augensicherheit von im Bestrahlungsraum befindlichen Personen maßgeblich ist, ist die maximal mögliche Helligkeit der Laserlinien begrenzt.

Dadurch kann sich in der Praxis ein relativ schlechter Kontrast zwischen den Laserlinien und den von der Kamera ebenfalls wahrgenommenen umgebenden Objekten ergeben. Zur Verbesserung der Laserlinienerkennung in den Kamerabildern können optische Bandpassfilter verwendet werden, die auf die jeweils verwendete Laserwellenlänge abgestimmt sind. Durch die Verwendung eines optischen Bandpassfilters können die die Laserlinien umgebenden Objekte zu einem gewissen Maße ausgeblendet werden.

Alternativ oder zusätzlich ist es möglich, dass die Auswerte- und Steuereinrichtung die jeweils angesteuerten Raumlaser derart ein- und ausschaltet, dass die Kameras in schneller Folge alternierend Bilder des jeweils von der Kamera erfassten Bereiches mit projizierter Laserlinie und ohne projizierte Laserlinie sehen. Unmittelbar aufeinanderfolgende Bilder können dann durch die Auswerte- und Steuereinrichtung jeweils voneinander subtrahiert werden, insbesondere pixelweise, so dass die Laserlinien als Differenz zweier unmittelbar aufeinanderfolgender Kamerabilder mit sehr hohem Kontrast hervortreten.

Bei mehreren projizierten und ausgewerteten Laserlinien können beispielsweise die Laserlinien selbst zeitlich versetzt zueinander projiziert werden, es kann also ein zeitliches Multiplexing erfolgen. Auf diese Weise kann sichergestellt werden, dass bestimmte Kameras immer nur eine Laserlinie zur Zeit sehen. Dies könnte auch erreicht werden, indem die verschiedenen Laserlinien von Lasern mit unterschiedlicher Wellenlänge projiziert werden und die jeweiligen Kameras beispielsweise durch Vorsehen geeigneter Filter nur eine Wellenlänge detektieren.

Insbesondere wenn zwei der verwendeten Raumlaser im Sollfall koplanar zueinander ausgerichtete fächerförmige Lichtebenen erzeugen, ist es darüber hinaus möglich, die Koplanarität dieser Lichtebenen mittels der erfindungsgemäß vorgesehenen Kameras zu überprüfen.

Nach einer weiteren Ausgestaltung können die Sollkoordinatenpunkte auf Grundlage einer der Bestrahlung vorangegangenen CT-Aufnahme des Patienten ermittelt und in der Speichereinrichtung der Steuer- und Auswerteeinrichtung abgelegt worden sein. Es ist dann weiter möglich, dass die Sollkoordinatenpunkte aus Schnittkoordinatenpunkten der im Rahmen der CT-Aufnahme ermittelten Oberfläche des Patienten mit mindestens einer durch das Zentrum eines zu bestrahlenden Gebiets des Patienten verlaufenden Ebene, vorzugsweise mit zwei oder drei senkrecht zueinander stehenden und sich im Zentrum eines zu bestrahlenden Gebiets des Patienten schneidenden Ebenen, ermittelt wurden.

Nach einer weiteren Ausgestaltung ist es möglich, dass die Sollkoordinatenpunkte ermittelt werden, nachdem der Patient vor einem Bestrahlungsvorgang mit einem bildgebenden Verfahren (Cone Beam CT, Ultraschall) in der vorgegebenen Bestrahlungsposition positioniert wurde, indem durch die Auswerte- und Steuereinrichtung auf Grundlage der von der Kamera detektierten Messwerte durch ein Echtzeit-Triangulationsverfahren Koordinatenpunkte entlang der auf die Oberfläche des Patienten projizierten Laserlinien ermittelt werden und die so ermittelten Koordinatenpunkte als Sollkoordinatenpunkte in der Speichereinrichtung der Steuer- und Auswerteeinrichtung abgelegt sind.

Das System kann weiterhin eine Anzeigeeinrichtung umfassen, die die während eines Bestrahlungsvorgangs ermittelten Ist-Koordinatenpunkte und optional auch die Sollkoordinatenpunkte in Echtzeit darstellt. Die Koordinatenpunkte können direkt oder in geeigneter Weise visualisiert dargestellt werden. Beispielsweise können gefittete Linien durch Koordinatenpunkte gelegt werden.

Die Auswerte- und Steuereinrichtung kann weiter dazu ausgebildet sein, bei einer unzulässigen Abweichung zwischen den ermittelten Koordinatenpunkten und den Sollkoordinatenpunkten ein Warnsignal auszugeben und/oder eine Korrektur der Patientenposition durch Ansteuern einer Bewegungssteuerung des Patiententisches vorzunehmen. Bei der Patientenpositionierung zu Beginn einer Bestrahlungsfraktion werden die jeweiligen Parameter eingestellt. Wird im Zuge der während der nachfolgenden Bestrahlung erfindungsgemäß durchgeführten Überwachung der Patientenposition eine unzulässige Abweichung festgestellt, kann zunächst ein Warnsignal ausgegeben werden. Das Warnsignal kann optisch und/oder akustisch und/oder haptisch sein. Ein Benutzer kann dann manuell Maßnahmen ergreifen, zum Beispiel den Patienten neu positionieren oder die Bestrahlung abbrechen. Natürlich ist es auch möglich, dass durch die Auswerte- und Steuereinrichtung eine Unterbrechung der Bestrahlung automatisch erfolgt, beispielsweise durch eine Not-Aus-Betätigung. Es ist aber auch eine vollautomatische Anpassung der Patientenposition möglich, indem die Auswerte- und Steuereinrichtung auf Grundlage der gemessenen Werte die Verfahrantriebe des Patiententisches so ansteuert, dass die gemessenen Ist-Koordinatenpunkte und die Sollkoordinatenpunkte wieder übereinstimmen. Es erfolgt somit also ein Tracking. Dabei kann ein 3D-Matching-Algorithmus zum Einsatz kommen, der an sich bekannt ist.

Die Auswerte- und Steuereinrichtung kann weiter dazu ausgebildet sein, durch die erfindungsgemäße Ermittlung der 3D-Koordinaten der Laserlinien eine Atembewegung oder eine anderweitige Bewegung des Patienten während eines Bestrahlungsvorgangs zu erfassen. Sie kann dann das Bestrahlungsgerät so ansteuern, dass eine Bestrahlung immer nur in einer vorgegebenen Atemposition oder anderweitigen Bewegungsposition des Patienten erfolgt. Es erfolgt also eine Echtzeitberücksichtigung des Atemhubs des Patienten durch eine geeignete Echtzeit-Positionsauswertung der insoweit flexiblen Patientenoberfläche. Dabei ist eine sogenannte 4D-Bestrahlung möglich. Es können auch 4D-CT-Daten herangezogen werden, um auf dieser Grundlage aus den gemessenen Koordinatenpunkten den Atemhub zu ermitteln.

Nach einer weiteren Ausgestaltung kann die Auswerte- und Steuereinrichtung dazu ausgebildet sein, die Koordinaten einer im Schnittpunkt eines Zentralstrahls des Bestrahlungsgeräts mit der Oberfläche des Patienten schneidenden Laserlinie zu ermitteln und daraus den Fokus-Haut-Abstand zu ermitteln. Der Fokus-Haut-Abstand ist definiert durch den Abstand des Fokus- oder Brennpunkts des Bestrahlungsgeräts zu der Patientenoberfläche entlang eines Vektors von dem Fokus- oder Brennpunkt des Bestrahlungsgeräts zum Isozentrum (in der Regel dem Nullpunkt des Koordinatensystems). Der Fokus-Haut-Abstand ist ein wichtiger Parameter in der Strahlentherapie.

Nach einer weiteren Ausgestaltung kann vorgesehen sein, dass mindestens einer der Raumlaser eine Laserlinie auf die Oberfläche des Patiententisches und/oder des Bestrahlungsgeräts in dem Bestrahlungsraum projiziert, wobei mindestens eine Kamera dazu ausgebildet ist, die von dem mindestens einen Raumlaser auf die Oberfläche des Patiententisches und/oder des Bestrahlungsgeräts projizierte Laserlinie zu detektieren, und wobei die Auswerte- und Steuereinrichtung dazu ausgebildet ist, während eines Bestrahlungsvorgangs auf Grundlage der von der Kamera detektierten Messwerte durch ein Echtzeit-Triangulationsverfahren die Koordinatenpunkte entlang der auf die Oberfläche des Patiententisches und/oder des Bestrahlungsgeräts projizierten Laserlinie zu ermitteln. Nach einer weiteren diesbezüglichen Ausgestaltung kann vorgesehen sein, dass die Auswerte- und Steuereinrichtung weiter dazu ausgebildet ist, die ermittelten Koordinatenpunkte entlang der auf die Oberfläche des Patiententisches und/oder des Bestrahlungsgeräts projizierten Laserlinie mit Sollkoordinatenpunkten zu vergleichen und bei einer unzulässigen Abweichung zwischen den ermittelten Koordinatenpunkten und den Sollkoordinatenpunkten ein Warnsignal, insbesondere ein Kollisionswarnsignal, auszugeben. Die Sollkoordinatenpunkte können beispielsweise im Zuge der Planung der Bestrahlungsbehandlung ermittelt und in der Speichereinrichtung gespeichert worden sein. Auch kann vorgesehen sein, dass die Auswerte- und Steuereinrichtung weiter dazu ausgebildet ist, die während des Bestrahlungsvorgangs ermittelten Koordinatenpunkte entlang der auf die Oberfläche des Patiententisches und/oder des Bestrahlungsgeräts projizierten Laserlinie in der Speichereinrichtung für eine Dokumentation des Bestrahlungsvorgangs zu speichern.

Wie erläutert, kann der Kopf des Bestrahlungsgeräts, also die Gantry, in einer festen Ebene um 360° gedreht werden. Bei dieser Rotationsbewegung wirkt sich das große Gewicht der Gantry unterschiedlich in verschiedenen Positionen auf die Genauigkeit der Drehbewegung aus. Wie eingangs erläutert, führen solche Ungenauigkeiten in der Drehbewegung zu unerwünschten Einflüssen auf die Bestrahlungsgenauigkeit. Das zusätzliche Gewicht einer in der Regel ausfahrbaren Röntgenröhre und des gegenüberliegenden Bilddetektors tragen zu weiteren Ungenauigkeiten bei. Der Patiententisch kann in der Regel sowohl Translationsbewegungen als auch Rotationsbewegungen durchführen. Je nach Position und Gewicht eines darauf befindlichen Pateinten sind Abweichungen von den jeweils vorgegebenen Positionen möglich, die sich wie eingangs erläutert ebenfalls unerwünscht auf das Bestrahlungsergebnis auswirken.

Bei den vorgenannten Ausgestaltungen erfolgt mittels der Raumlaser weiterhin eine Echtzeitbestimmung der Position des Bestrahlungsgeräts und/oder des Patiententisches. Auch weitere im Bestrahlungsraum vorhandene Objekte können auf diese Weise überwacht werden und es kann beispielsweise eine Kollision verhindert werden. Die durch das Triangulationsverfahren bestimmten Koordinaten der jeweils projizierten Linien geben dabei allein noch nicht notwendigerweise eine ausreichende Auskunft über die Position des Objektes im Raum. Aus diesem Grund müssen beispielsweise die 3D-Koordinaten der Punkte der Oberfläche des zu überwachenden Objektes, beispielsweise einer Gantry, bekannt sein, insbesondere in Form von 3D-CAD-Daten oder aus Anfangs-Einmessungen. Durch einen in der Software der Auswerte- und Steuereinrichtung hinterlegten mathematischen Suchalgorithmus wird beispielsweis der Gantry-Drehwinkel bestimmt, bei dem die theoretisch bestimmten Koordinaten virtueller Laserprojektionslinien die gleichen Werte besitzen wie die durch das Triangulationsverfahren messtechnisch bestimmten Koordinaten der Laserprojektionslinien. Je mehr projizierte Linien ausgewertet werden, desto schneller kann die Positionsbestimmung erfolgen. Durch eine Auswertung der bekannten Ausgangsposition der Objekte im Bestrahlungsraum sowie deren ebenfalls bekannte 3D-Bewegungsfreiheitsgrade und 3D-Oberflächen kann durch die Software der Auswerte- und Steuereinrichtung mittels an sich bekannter mathematischer Verfahren die Position der Objekte im Bestrahlungsraum in Echtzeit bestimmt werden.

Die messtechnisch ermittelte Position weiterer Objekte im Bestrahlungsraum über den Patienten hinaus kann insbesondere auch bei der Dokumentation in der Speichereinrichtung berücksichtigt werden, so dass die jeweils bei einer Bestrahlungsfraktion von dem Patienten effektiv erhaltene Bestrahlungsdosis präzise ermittelt werden kann und beispielsweise bei der Einstellung weiterer Bestrahlungsvorgänge berücksichtigt werden kann.

Ein weiteres Problemfeld ist die Zuordnung der gemessenen 3D-Koordinaten zu einem bestimmten Objekt, also die Frage, ob die Kamera Laserlinienkoordinaten auf dem zu messenden Objekt oder auf einem anderen Objekt misst. Dieses Problemfeld kann auf zwei Weisen gelöst werden. Bei einer ersten Alternative kann das zu vermessende Objekt im Rahmen einer Kalibrierung in unterschiedlichste Positionen verfahren werden und es können jeweils die entsprechend projizierten Laserlinien aufgenommen und gespeichert werden. Bei der anschließenden Messung kann dann ein Vergleich der gemessenen Linien mit den gespeicherten Linien erfolgen und durch ein Matching-Verfahren anhand der empirisch durchgeführten Zuordnung bestimmter Positionen des Objekts zu bestimmten Laserlinien auf die während der Messung vorliegende Position rückgeschlossen werden. Nach einer zweiten Alternative können unterschiedliche Oberflächenbeschaffenheiten der Objekte, beispielsweise unterschiedliche Reflektivitäten, ausgewertet werden. Auch denkbar ist das Anbringen von Kontrastmarkierungen unterschiedlicher Art an unterschiedlichen Objekten um im Rahmen der Auswertung die unterschiedlichen Objekte diskriminieren zu können.

Nach einer weiteren Ausgestaltung kann vorgesehen sein, dass mindestens vier Raumlaser vorgesehen sind, wobei von den mindestens vier Raumlasern zwei auf gegenüberliegenden Seiten des Patiententisches angeordnet sind und jeweils eine seitliche horizontale Laserlinie und eine Transversallinie auf einen auf dem Patiententisch liegenden Patienten projizieren, wobei von den mindestens vier Raumlasern weiterhin mindestens zwei oberhalb des Patiententisches angeordnet sind, von denen einer zumindest eine Transversallinie auf einen auf dem Patiententisch liegenden Patienten projiziert, und von denen einer eine Longitudinallinie auf einen auf dem Patiententisch liegenden Patienten projiziert.

Die seitlich des Patiententisches angeordneten ersten und zweiten Raumlaser erzeugen also jeweils zwei fächerförmige und zueinander orthogonale Laserlichtebenen. Die von diesen zwei einander gegenüberliegenden und auf den beiden Längsseiten des Patiententisches angeordneten Raumlasern ausgesandten Laserlichtebenen sollen jeweils paarweise koplanar zueinander sein. Der oberhalb des Patiententisches angeordnete, die Transversallinie erzeugende dritte Raumlaser kann neben der die Transversallinie erzeugenden fächerförmigen Laserlichtebene zusätzlich eine zu dieser orthogonale fächerförmige Laserlichtebene erzeugen, die (wie die Laserlichtebene des oberhalb des Patiententisches angeordneten vierten Raumlasers) ebenfalls eine Longitudinallinie auf dem Patientenkörper erzeugt. Die Laserlichtebenen dieser drei Raumlaser sollen sich im Isozentrum des Bestrahlungsgeräts schneiden. Diese Raumlaser erzeugen drei Linien auf der Patientenoberfläche, eine longitudinale, eine transversale und eine von jeder Seite horizontal seitliche (koronale) Linie. Dadurch entstehen drei Kreuze auf der Patientenoberfläche (seitlich links und rechts sowie oben). Die Ursprungspunkte der ersten drei Raumlaser (links, rechts und oben) können koplanar sein. Diese drei Raumlaser sind dann in einer senkrecht zur Längsachse des Patiententisches verlaufenden Ebene angeordnet. Der vierte Raumlaser kann ebenso wie der dritte Raumlaser oberhalb des Pateiententisches angeordnet sein. Dieser vierte Raumlaser, der insbesondere nur eine Laserlinie erzeugt, ist mit seinem Ursprungspunkt nicht in der gemeinsamen senkrecht zur Längsachse des Patiententisches verlaufenden Ebene der übrigen Raumlaser angeordnet. Er ist vielmehr in Längsrichtung des Patiententisches versetzt angeordnet. Die Laserlichtebene des vierten Raumlasers verläuft aber ebenfalls durch den Schnittpunkt der von den übrigen Raumlasern erzeugten Laserlichtebenen. Außerdem liegt die von diesem vierten Raumlaser erzeugte Laserlichtebene in der gleichen Ebene wie die die Longitudinallinie erzeugende Laserlichtebene des dritten Raumlasers (Deckenlaser). Aufgrund ihrer großen Auffächerung projizieren die Raumlaser dabei jeweils auch Laserlinien auf den Patienten umgebende Objekte im Bestrahlungsraum, wie Bestrahlungsgerät und Patiententisch. Es soll in jeder Position des Bestrahlungsgeräts ein Kreuz auf den Patienten projizierbar sein. Da die Gantry in ihrer oberen (Null-)Position den dritten Raumlaser abschattet, übernimmt in diesem Fall der vierte Raumlaser die Projektion der Longitudinallinie, so dass trotzdem ein Kreuz auf der Oberseite des Patientenkörpers abgebildet werden kann.

Es ist möglich, dass die seitlichen ersten und zweiten Raumlaser und/oder der obere dritte Raumlaser jeweils eine Laserquelle umfassen, die über geeignete Optiken die beiden zueinander orthogonalen Laserlichtebenen erzeugt. Es ist aber auch möglich, dass die seitlichen ersten und zweiten Raumlaser und/oder der obere dritte Raumlaser jeweils zwei Laserquellen umfassen, von denen jeweils eine Laserquelle jeweils eine der orthogonalen Laserlichtebenen erzeugt. In diesem Fall können die beiden Laserquellen in einem gemeinsamen Gehäuse angeordnet sein oder auch räumlich getrennt in separaten Gehäusen.

Nach einer weiteren Ausgestaltung können mindestens zwei insbesondere hochauflösende Kameras vorgesehen sein, die jeweils dazu ausgebildet sind, die von mindestens zwei Raumlasern projizierten Laserlinien zu detektieren. Es kann sich beispielsweise um CCD-Kameras oder ähnliche optische Sensoren handeln. Natürlich können auch mehr als zwei solcher Kameras vorgesehen sein.

Das erfindungsgemäße System kann zur Durchführung des beschriebenen Verfahrens geeignet sein. Entsprechend kann das beschriebene Verfahren mit dem erfindungsgemäßen System durchgeführt werden.

Ausführungsbeispiele der Erfindung werden nachfolgend anhand von Figuren näher erläutert. Es zeigen schematisch:
- Fig. 1: ein erfindungsgemäßes System in einem ersten Betriebszustand,
- Fig. 2: das System aus Fig. 1 in einem zweiten Betriebszustand,
- Fig. 3: Bestandteile des Systems aus Fig. 1 in einem dritten Betriebszustand,
- Fig. 4: Bestandteile des in Fig. 1 gezeigten Systems in einem vierten Betriebszustand,
- Fig. 5: Beispieldarstellungen bei korrekter Patientenpositionierung, und
- Fig. 6: die Beispieldarstellungen aus Fig. 5 bei nicht korrekter Patientenpositionierung.

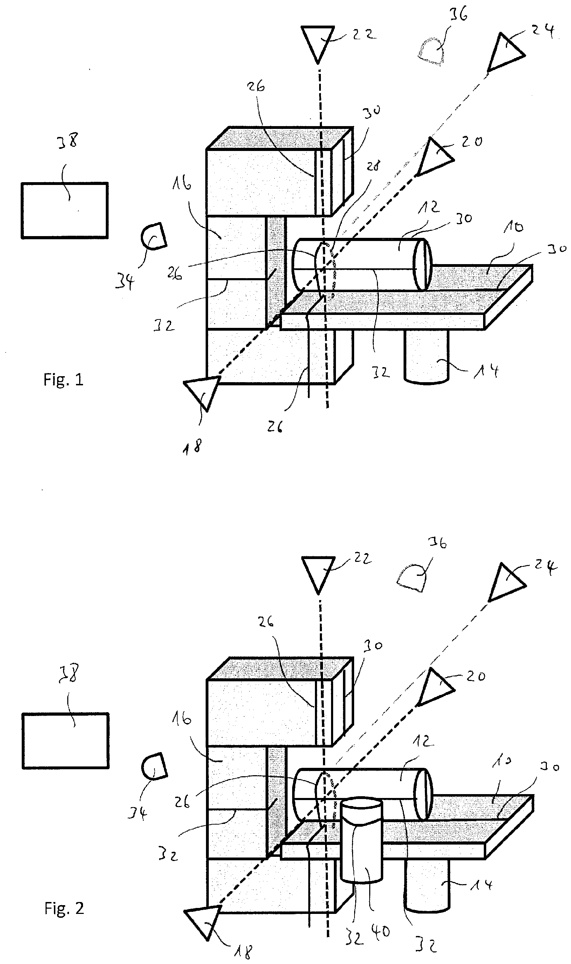

Soweit nichts anderes angegeben ist, bezeichnen in den Figuren gleiche Bezugszeichen gleiche Gegenstände. Das in Fig. 1 sehr schematisch dargestellte erfindungsgemäße System umfasst einen Patiententisch 10, auf dem ein in Fig. 1 durch einen Zylinder 12 veranschaulichter Patient gelagert werden kann. Der Patiententisch 10 steht über einen Fuß 14 auf dem Untergrund auf. Mittels nicht dargestellter Antriebe kann der Patiententisch 10 sowohl translatorisch in Längsrichtung und Querrichtung als auch rotatorisch um seine Längsachse und seine Querachse verfahren werden. Das System besitzt darüber hinaus einen Linearbeschleuniger als Bestrahlungsgerät 16, der in an sich bekannter Weise um 360° um den Patiententisch 10 drehbar ist.

Darüber hinaus umfasst das erfindungsgemäße System mehrere in dem Bestrahlungsraum fest angeordnete Raumlaser. In dem gezeigten Beispiel ist auf jeder Längsseite des Patiententisches 10 jeweils ein erster und zweiter seitlicher Raumlaser 18, 20 an der Wand des Bestrahlungsraums angebracht. Darüber hinaus befinden sich oberhalb des Patiententisches 10 ein dritter Raumlaser 22 und ein vierter Raumlaser 24, die in dem gezeigten Beispiel an der Decke des Bestrahlungsraums befestigt sind. Jeder der Raumlaser 18, 20, 22, 24 kann senkrecht zu zumindest einer von ihm projizierten Laserlinie verfahrbar sein. Die beiden seitlich angeordneten ersten und zweiten Raumlaser 18, 20 projizieren einerseits eine vertikale Laserlinie 26, 28 auf den Patientenkörper 12 und darüber hinaus auf den Patiententisch 10 und das Bestrahlungsgerät 16. Die von den seitlichen Raumlasern 18, 20 hierzu erzeugten fächerförmigen Laserlichtebenen liegen koplanar zueinander. Der obere dritte Raumlaser 22 erzeugt ebenfalls eine koplanar hierzu verlaufende Lichtebene und bildet so gemeinsam mit den seitlichen Raumlasern 18, 20 die sogenannte Transversallinie. Dabei liegt die die Transversallinie erzeugende Laserlichtebene des dritten Raumlaser 22 koplanar zu den die vertikalen Laserlinien 26, 28 erzeugenden Laserlichtebenen der seitlichen Raumlaser 18, 20. Darüber hinaus wird von den seitlich angeordneten ersten und zweiten Raumlasern 18, 20 jeweils eine horizontale Laserlinie 32 auf den Patientenkörper 12 sowie das Bestrahlungsgerät 16 projiziert. Auch die von den seitlichen ersten und zweiten Raumlasern 18, 20 hierzu erzeugten fächerförmigen Laserlichtebenen liegen koplanar zueinander. Auch der obere dritte Raumlaser 22 erzeugt eine zweite Laserlichtebene, die in dem gezeigten Beispiel eine Longitudinallinie 30 auf dem Patientenkörper 12 und auf dem Patiententisch 10 sowie dem Bestrahlungsgerät 16 erzeugt. Der obere vierte Raumlaser 24 projiziert gemeinsam mit dem dritten Raumlaser 22 die Longitudinallinie 30 auf den Patientenkörper 12 und auf den Patiententisch 10 sowie das Bestrahlungsgerät 16. Die die Longitudinallinie 30 bildenden Laserlichtebenen der dritten und vierten Raumlaser 22, 24 liegen koplanar zueinander. Erkennbar sind die Raumlaser 18, 20, 22 mit ihrem Ursprungspunkt in derselben senkrecht zur Längsachse des Patiententisches 10 verlaufenden Ebene angeordnet. Der vierte Raumlaser 24 ist dagegen in Längsrichtung des Patiententisches 10 versetzt zu den anderen Raumlasern 18, 20, 22 angeordnet. Dadurch kann in jeder Drehposition des Bestrahlungsgeräts 16 ein Laserlinienkreuz auf den Patientenkörper 12 projiziert werden. Die Laserlichtebenen der Raumlaser 18, 20, 22, 24 schneiden sich im Isozentrum des Bestrahlungsgeräts 16.

Das erfindungsgemäße System umfasst darüber hinaus in dem dargestellten Beispiel zwei hochauflösende Kameras 34, 36. Bei den Kameras kann es sich beispielsweise um CCD-Kameras handeln. Die Kameras 34, 36 sind so ausgerichtet, dass sie gemeinsam sämtliche von den Raumlasern 18, 20, 22, 24 projizierten Laserlinien detektieren können. Bei dem Bezugszeichen 38 ist eine Auswerte- und Steuereinrichtung des erfindungsgemäßen Systems gezeigt, die über geeignete nicht näher dargestellte Leitungen mit den Kameras 34, 36 und den Raumlasern 18, 20, 22, 24 verbunden ist. Die Auswerte- und Steuereinrichtung 38 kann die Raumlaser 18, 20, 22, 24 in der oben erläuterten Weise zum Erzeugen einer Laserlinie ansteuern. Außerdem kann die Auswerte- und Steuereinrichtung 38 die von den Kameras 34, 36 jeweils aufgenommenen Messdaten auslesen. Auf dieser Grundlage ermittelt die Auswerte- und Steuereinrichtung 38 während eines Bestrahlungsvorgangs durch ein Echtzeit-Triangulationsverfahren die 3D-Koordinatenpunkte entlang der auf die Oberfläche des Patientenkörpers 12 projizierten Laserlinien und vergleicht diese mit Sollkoordinatenpunkten. Dies kann in der oben erläuterten Weise geschehen. Auf dieser Grundlage kann die Auswerte- und Steuereinrichtung 38 weitere Maßnahmen ergreifen, beispielsweise eine unzulässige Abweichung in der oben erläuterten Weise visualisieren oder den Patiententisch 10 in geeigneter Weise über nicht dargestellte Leitungen ansteuern, um den Patientenkörper 12 neu zu positionieren.

Außerdem kann die Auswerte- und Steuereinrichtung 38 ebenfalls in der oben erläuterten Weise die auf die Oberfläche des Patiententisches 10 und/oder auf die Oberfläche des Bestrahlungsgeräts 16 projizierten Laserlinien hinsichtlich ihrer 3D-Koordinatenpunkte auswerten. Auch hier kann eine unzulässige Abweichung festgestellt werden und es können die bereits erläuterten Gegenmaßnahmen ergriffen werden.

Darüber hinaus umfasst die Auswerte- und Steuereinrichtung 38 eine Speichereinrichtung, in die die während eines Bestrahlungsvorgangs ermittelten Koordinatenpunkte für eine Dokumentation des Bestrahlungsvorgangs und gegebenenfalls eine Anpassung weiterer Bestrahlungsvorgänge gespeichert werden.

In dem in Fig. 2 gezeigten Betriebszustand ist ein weiteres Objekt 40 in dem Bestrahlungsraum angeordnet, wobei in diesem Fall die Laserlinie 32 sich auch über dieses Objekt 40 erstreckt. Dies kann wiederum durch Ermittlung der 3D-Koordinatenpunkte von der Auswerte- und Steuereinrichtung 38 festgestellt werden und es kann beispielsweise eine Kollisionswarnung erstellt werden, falls zum Beispiel das Bestrahlungsgerät 16 im Zuge seiner Rotation mit dem Objekt 40 kollidieren würde. Auch kann durch die Auswerte- und Steuereinrichtung 38 notfalls die Bestrahlung unterbrochen werden, um eine Kollision zu verhindern.

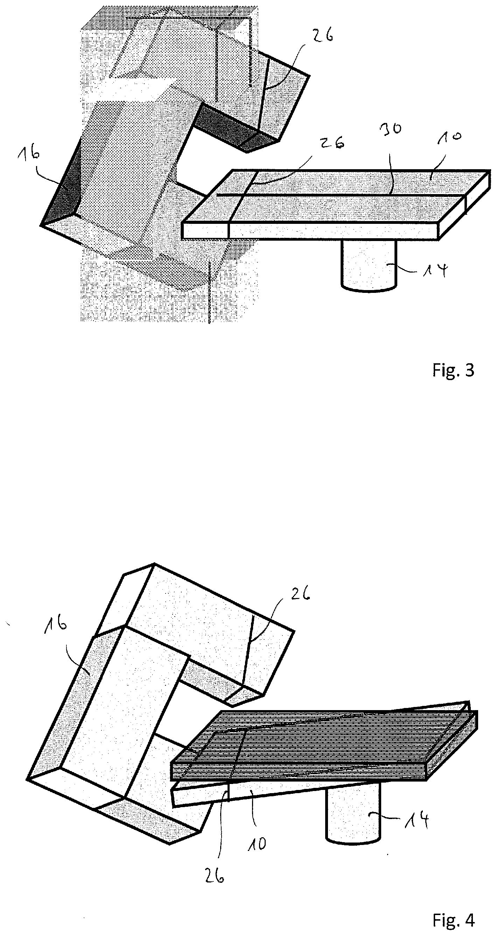

Anhand der Fig. 3 soll in stark vereinfachter Darstellung erläutert werden, wie sich eine Drehung des Bestrahlungsgeräts 16 aus der in Fig. 3 teiltransparent dargestellten Ausgangslage auswirkt. Dabei ist zu erkennen, dass insbesondere die Laserlinie 26 sich hinsichtlich ihrer Form und ihrer Position im Raum ändert. Dies kann durch die Auswerte- und Steuereinrichtung 38 zusammen mit den Raumlasern 18, 20, 22, 24 und den Kameras 34, 36 detektiert werden. Auf dieser Grundlage kann in der oben erläuterten Weise auf den jeweiligen Drehwinkel des Bestrahlungsgeräts 16 rückgeschlossen werden. Auch kann eine durch das Gewicht des Bestrahlungsgeräts 16 verursachte Abweichung von der kreisförmigen Bewegungsrichtung festgestellt werden und in der Speichereinrichtung für eine Dokumentation gespeichert werden.

In Fig. 4 ist die Auswirkung einer Lageveränderung des Patiententisches 10 gegenüber der in Fig. 4 teiltransparent dargestellten Ausgangslage gezeigt. Dadurch kommt es wiederum zu einer Veränderung der Linienform und -position insbesondere der Laserlinie 26 und auch der in Fig. 4 nicht dargestellten Laserlinie 30, die in der erfindungsgemäßen Weise wie erläutert festgestellt werden kann. Auch diese Abweichung kann in der Speichereinrichtung dokumentiert werden.

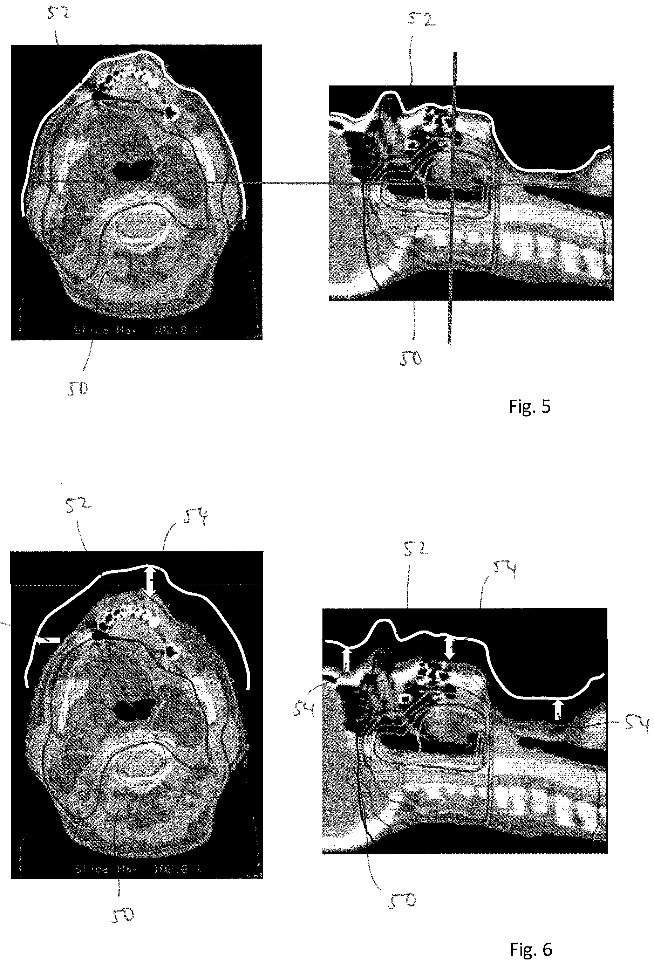

Anhand der Fig. 5 und 6 soll schematisch die Überwachung der Patientenposition erläutert werden. Dabei ist jeweils in der linken und rechten Bildhälfte ein aus einer CT-Aufnahme stammendes Schnittbild 50 eines Kopfes eines Patienten dargestellt. Bei dem Bezugszeichen 52 ist jeweils in weiß eine mit dem beschriebenen Verfahren bzw. dem erfindungsgemäßen System ermittelte Koordinatenlinie dargestellt, die die real gemessene Oberfläche des Patientenkopfes während der Bestrahlung links in einem Transversalschnitt und rechts in einem Longitudinalschnitt zeigt. Die Sollkoordinaten für die Laserlinien 52 werden jeweils durch die Oberfläche des Patientenkopfes im CT-Schnittbild 50 vorgegeben. Durch die erfindungsgemäße Auswerte- und Steuereinrichtung 38 werden die gemessenen Koordinatenpunkte entlang der Linien 52 mit diesen Sollkoordinaten verglichen. In der Fig. 5 ist eine korrekte Position des Patienten nach den Sollkoordinaten gegeben, insbesondere liegt der Patientenkopf mit dem zu bestrahlenden Gebiet im Isozentrum des Bestrahlungsgeräts 16. In Fig. 6 ist zu erkennen, dass die gemessenen Koordinatenpunkte entlang der Laserlinien 52 von den durch die Oberfläche der CT-Schnittbilder gebildeten Sollkoordinaten abweichen, insbesondere liegt der Patient in dem in Fig. 6 gezeigten Beispiel zu hoch. Die Abweichung ist in Fig. 6 durch die Pfeile 54 veranschaulicht. Durch eine Verfahrbewegung des Patiententisches 10, angesteuert beispielsweise automatisch durch die Auswerte- und Steuereinrichtung 38, kann der Patientenkörper nach unten verfahren werden, bis die in Echtzeit gemessenen Koordinaten der Laserlinien 52 mit den Sollkoordinaten aus den CT-Schnittbildern übereinstimmen.

## Patentansprüche

1. System zum Ermitteln der Position von Objekten in einem Bestrahlungsraum für eine Strahlentherapie, umfassend mehrere in dem Bestrahlungsraum angeordnete Raumlaser (18, 20, 22, 24), die jeweils dazu ausgebildet sind, für die Patientenpositionierung mindestens eine Laserlinie auf die Oberfläche eines auf einem Patiententisch (10) in dem Bestrahlungsraum befindlichen Patienten zu projizieren, weiter umfassend mindestens eine Kamera (34, 36), die dazu ausgebildet ist, mindestens eine von mindestens einem der Raumlaser (18, 20, 22, 24) auf die Oberfläche des Patienten projizierte Laserlinie zu detektieren, und umfassend eine Auswerte- und Steuereinrichtung (38), die dazu ausgebildet ist, während eines Bestrahlungsvorgangs auf Grundlage der von der Kamera (34, 36) detektierten Messwerte durch ein Echtzeit-Triangulationsverfahren die Koordinatenpunkte entlang der auf die Oberfläche des Patienten projizierten Laserlinie zu ermitteln und für die Patientenpositionierung mit Sollkoordinatenpunkten zu vergleichen, wobei die Steuer- und Auswerteeinrichtung weiterhin eine Speichereinrichtung umfasst, in der die während des Bestrahlungsvorgangs ermittelten Koordinatenpunkte für eine Dokumentation des Bestrahlungsvorgangs gespeichert werden, **dadurch gekennzeichnet, dass** mindestens einer der Raumlaser (18, 20, 22, 24) eine Laserlinie auf die Oberfläche des Bestrahlungsgeräts (16) in dem Bestrahlungsraum projiziert, wobei mindestens eine Kamera (34, 36) dazu ausgebildet ist, die von dem mindestens einen Raumlaser (18, 20, 22, 24) auf die Oberfläche des Bestrahlungsgeräts (16) projizierte Laserlinie zu detektieren, und wobei die Auswerte- und Steuereinrichtung (38) dazu ausgebildet ist, während eines Bestrahlungsvorgangs auf Grundlage der von der Kamera (34, 36) detektierten Messwerte durch ein Echtzeit-Triangulationsverfahren die Koordinatenpunkte entlang der auf die Oberfläche des Bestrahlungsgeräts (16) projizierten Laserlinie zu ermitteln, wobei die Auswerte- und Steuereinrichtung (38) weiter dazu ausgebildet ist, die während des Bestrahlungsvorgangs ermittelten Koordinatenpunkte entlang der auf die Oberfläche des Bestrahlungsgeräts (16) projizierten Laserlinie in der Speichereinrichtung für eine Dokumentation des Bestrahlungsvorgangs zu speichern.

2. System nach Anspruch 1, wobei die Sollkoordinatenpunkte auf Grundlage einer der Bestrahlung vorangegangenen CT-Aufnahme des Patienten oder eines anderen geeigneten Referenzbildverfahren ermittelt wurden und in der Speichereinrichtung der Steuer- und Auswerteeinrichtung abgelegt sind.

3. System nach Anspruch 2, wobei die Sollkoordinatenpunkte aus Schnittkoordinatenpunkten der im Rahmen der CT-Aufnahme oder eines anderen geeigneten Referenzbildverfahren ermittelten Oberfläche des Patienten mit mindestens einer durch das Zentrum eines zu bestrahlenden Gebiets des Patienten verlaufenden Ebene, vorzugsweise mit zwei oder drei senkrecht zueinander stehenden und sich im Zentrum eines zu bestrahlenden Gebiets des Patienten schneidenden Ebenen, ermittelt wurden.

4. System nach einem der vorhergehenden Ansprüche, wobei die Sollkoordinatenpunkte ermittelt wurden, nachdem der Patient vor einem Bestrahlungsvorgang mit einem bildgebenden Verfahren mit seinem zu bestrahlenden Gebiet im Isozentrum des Bestrahlungsgeräts positioniert wurde, indem durch die Auswerte- und Steuereinrichtung (38) auf Grundlage der von der Kamera (34, 36) detektierten Messwerte durch ein Echtzeit-Triangulationsverfahren Koordinatenpunkte entlang der auf die Oberfläche des Patienten projizierten Laserlinie ermittelt wurden und die so ermittelten Koordinatenpunkte als Sollkoordinatenpunkte in der Speichereinrichtung der Steuer- und Auswerteeinrichtung abgelegt sind.

5. System nach einem der vorhergehenden Ansprüche, weiter umfassend eine Anzeigeeinrichtung, die die während eines Bestrahlungsvorgangs ermittelten Koordinatenpunkte und optional auch die Sollkoordinatenpunkte in Echtzeit darstellt.

6. System nach einem der vorhergehenden Ansprüche, wobei die Auswerte- und Steuereinrichtung (38) weiter dazu ausgebildet ist, bei einer unzulässigen Abweichung zwischen den ermittelten Koordinatenpunkten und den Sollkoordinatenpunkten ein Warnsignal auszugeben und/oder eine Korrektur der Patientenposition durch Ansteuern einer Bewegungssteuerung des Patiententisches (10) vorzunehmen.

7. System nach einem der vorhergehenden Ansprüche, wobei die Auswerte- und Steuereinrichtung (38) weiter dazu ausgebildet ist, eine Atembewegung oder eine anderweitige Bewegung des Patienten während eines Bestrahlungsvorgangs zu erfassen.

8. System nach Anspruch 7, wobei die Auswerte- und Steuereinrichtung (38) weiter dazu ausgebildet ist, das Bestrahlungsgerät so anzusteuern, dass eine Bestrahlung immer nur in einer vorgegebenen Atemposition oder anderweitigen Bewegungsposition des Patienten erfolgt.

9. System nach einem der vorhergehenden Ansprüche, wobei die Auswerte- und Steuereinrichtung (38) weiter dazu ausgebildet ist, die Punktkoordinaten zweier sich im Schnittpunkt eines Zentralstrahls des Bestrahlungsgeräts mit der Oberfläche des Patienten schneidenden Laserlinien zu ermitteln und daraus den Fokus-Haut-Abstand zu bestimmen.

10. System nach einem der vorhergehenden Ansprüche, wobei mindestens einer der Raumlaser (18, 20, 22, 24) eine Laserlinie auf die Oberfläche des Patiententisches (10) in dem Bestrahlungsraum projiziert, wobei mindestens eine Kamera (34, 36) dazu ausgebildet ist, die von dem mindestens einen Raumlaser (18, 20, 22, 24) auf die Oberfläche des Patiententisches (10) projizierte Laserlinie zu detektieren, und wobei die Auswerte- und Steuereinrichtung (38) dazu ausgebildet ist, während eines Bestrahlungsvorgangs auf Grundlage der von der Kamera (34, 36) detektierten Messwerte durch ein Echtzeit-Triangulationsverfahren die Koordinatenpunkte entlang der auf die Oberfläche des Patiententisches (10) projizierten Laserlinie zu ermitteln.

11. System nach Anspruch 10, wobei die Auswerte- und Steuereinrichtung (38) weiter dazu ausgebildet ist, die ermittelten Koordinatenpunkte entlang der auf die Oberfläche des Patiententisches (10) und/oder des Bestrahlungsgeräts (16) projizierten Laserlinie mit Sollkoordinatenpunkten zu vergleichen und bei einer unzulässigen Abweichung zwischen den ermittelten Koordinatenpunkten und den Sollkoordinatenpunkten ein Warnsignal, insbesondere ein Kollisionswarnsignal, auszugeben.

12. System nach einem der Ansprüche 10 oder 11, wobei die Auswerte- und Steuereinrichtung (38) weiter dazu ausgebildet ist, die während des Bestrahlungsvorgangs ermittelten Koordinatenpunkte entlang der auf die Oberfläche des Patiententisches (10) projizierten Laserlinie in der Speichereinrichtung für eine Dokumentation des Bestrahlungsvorgangs zu speichern.

13. System nach einem der vorhergehenden Ansprüche, wobei mindestens vier Raumlaser (18, 20, 22, 24) vorgesehen sind, wobei von den mindestens vier Raumlasern (18, 20, 22, 24) zwei auf gegenüberliegenden Seiten des Patiententisches (10) angeordnet sind und jeweils eine seitliche horizontale Laserlinie und eine Transversallinie auf einen auf dem Patiententisch (10) liegenden Patienten projizieren, wobei von den mindestens vier Raumlasern (18, 20, 22, 24) weiterhin mindestens zwei oberhalb des Patiententisches (10) angeordnet sind, von denen einer zumindest eine Transversallinie auf einen auf dem Patiententisch (10) liegenden Patienten projiziert, und von denen einer eine Longitudinallinie auf einen auf dem Patiententisch (10) liegenden Patienten projiziert.

14. System nach Anspruch 13, wobei die seitlichen Raumlaser (18, 20) jeweils zwei Laserquellen umfassen, von denen die eine die seitliche horizontale Laserlinie und die andere die Transversallinie auf den auf dem Patiententisch (10) liegenden Patienten projiziert.

15. System nach Anspruch 13 oder Anspruch 14, wobei mindestens zwei Kameras (34, 36) vorgesehen sind, die jeweils dazu ausgebildet sind, die von mindestens zwei Raumlasern (18, 20) projizierten Laserlinien zu detektieren.

## Claims

1. A system for determining the position of objects in an irradiation room for radiation therapy, comprising a plurality of room lasers (18, 20, 22, 24) arranged in the irradiation room which are each designed to project at least one laser line on the surface of a patient located in the irradiation room on a patient table (10) for patient positioning, moreover comprising at least one camera (34, 36) that is designed to detect at least one laser line projected onto the surface of the patient by at least one of the room lasers (18, 20, 22, 24), and comprising an evaluation and control apparatus (38) that is designed to ascertain by a real-time triangulation method the coordinate points along the laser line projected onto the surface of the patient during an irradiation process based on the measured values detected by the camera (34, 36) and to compare them with target coordination points for patient positioning, wherein the control and evaluation apparatus moreover comprises a memory apparatus in which the coordinate points determined during the irradiation process are saved for documenting the irradiation process, **characterized in that** at least one of the room lasers (18, 20, 22, 24) projects a laser line onto the surface of the radiation device (16) in the irradiation room, wherein at least one camera (34, 36) is designed to detect the laser line projected by the at least one room laser (18, 20, 22, 24) on the surface of the radiation device (16), and wherein the evaluation and control device (38) is designed to ascertain by a real-time triangulation method the coordinate points along the laser line projected onto the surface of the radiation device (16) during an irradiation process based on the measured values detected by the camera (34, 36), wherein the evaluation and control apparatus (38) is further designed to save the coordinate points determined during the irradiation process along the laser line projected onto the surface of the radiation device (16) in the memory apparatus for documenting the irradiation process.

2. The system according to claim 1, wherein the target coordinate points were determined based on a CT image of the patient or on another suitable reference image process performed before the radiation procedure and are stored in the memory device of the evaluation and control apparatus.

3. The system according to claim 2, wherein the target coordinate points were determined from intersection coordinate points of the surface of the patient determined within the framework of the CT image or another suitable reference image process with at least one plane progressing through the center of an area of the patient to be irradiated, preferably with two or three planes located perpendicular to each other and intersecting in the center of an area of the patient to be irradiated.

4. The system according to one of the previous claims, wherein the target coordinate points were determined after the patient was positioned with his area to be irradiated in the isocenter of the radiation device before a radiation procedure with an imaging process, in that coordinate points along the laser line projected on the surface of the patient were determined by the evaluation and control apparatus (38) based on the measurement values detected by the camera (34, 36) through a real-time triangulation process, and the coordinate points determined in this manner are saved as target coordinate points in the memory device of the evaluation and control apparatus.

5. The system according to one of the previous claims, further comprising a display apparatus, which shows the coordinate points determined during a radiation procedure as well as, optionally, the target coordinate points in real time.

6. The system according to one of the previous claims, wherein the evaluation and control apparatus (38) is further designed to emit a warning signal in the case of an impermissible deviation between the determined coordinate points and the target coordinate points and/or to perform a correction of the patient position by activating a movement control of the patient table (10).

7. The system according to one of the previous claims, wherein the evaluation and control apparatus (38) is further designed to capture a breathing movement or another type of movement of the patient during a radiation procedure.

8. The system according to claim 7, wherein the evaluation and control apparatus (38) is further designed to control the radiation device such that radiation only takes place in a specified breathing position or other movement position of the patient.

9. The system according to one of the previous claims, wherein the evaluation and control apparatus (38) is further designed to determine the point coordinates of two laser lines intersecting at the intersection of a central beam of the radiation device with the surface of the patient and to determine the focus-skin distance from this.

10. The system according to one of the previous claims, wherein at least one of the room lasers (18, 20, 22, 24) projects a laser line onto the surface of the patient table (10) in the irradiation room, wherein at least one camera (34, 36) is designed to detect the laser line projected onto the surface of the patient table (10) and/or radiation device by the at least one room laser (18, 20, 22, 24), and wherein the evaluation and control apparatus (38) is designed to determine the coordinate points along the laser line projected onto the surface of the patient table (10) during a radiation procedure based on the measurement values detected by the camera (34, 36) through a real-time triangulation process.

11. The system according to claim 10, wherein the evaluation and control apparatus (38) is further designed to compare the determined coordinate points along the laser line projected onto the surface of the patient table (10) and/or radiation device (16) with target coordinate points and to emit a warning signal, in particular a collision warning signal, in the case of an impermissible deviation between the determined coordinate points and the target coordinate points.

12. The system according to one of claims 10 or 11, wherein the evaluation and control apparatus (38) is further designed to save the coordinate points along the laser line projected onto the surface of the patient table (10) determined during the radiation procedure in the memory device for documentation of the radiation procedure.

13. The system according to one of the previous claims,wherein at least four room lasers (18, 20, 22, 24) are provided, wherein, of the at least four room lasers (18, 20, 22, 24), two are arranged on opposite-lying sides of the patient table (10) and project respectively one lateral horizontal laser line and one transverse line onto a patient lying on the patient table (10), wherein furthermore, of the at least four room lasers (18, 20, 22, 24), at least two are arranged above the patient table (10), one of which projects at least one transversal line onto a patient lying on the patient table (10), and one of which projects a longitudinal line onto a patient lying on the patient table (10).

14. The system according to claim 13, wherein the lateral room lasers (18, 20) comprise respectively two laser sources, one of which projects the lateral horizontal laser line and the other the transversal line onto the patient lying on the patient table (10).

15. The system according to claim 13 or 14, wherein at least two cameras (34, 36) are provided which are each designed to detect the laser lines projected by at least two room lasers (18, 20).

## Revendications

1. Système destiné à déterminer la position d'objets dans une chambre d'irradiation pour une radiothérapie, comprenant plusieurs lasers spatiaux (18, 20, 22, 24) disposés dans la chambre d'irradiation, qui sont respectivement conçus de manière à projeter au moins une ligne laser à la surface d'un patient se trouvant sur une table de traitement (10) dans la chambre d'irradiation afin de positionner le patient, comprenant encore au moins une caméra (34, 36), qui est conçue de manière à détecter au moins une ligne laser projetée à la surface du patient par au moins l'un des lasers spatiaux (18, 20, 22, 24), et comprenant un dispositif d'analyse et de commande (38), qui est conçu de manière à déterminer les points de coordonnées le long de la ligne laser projetée à la surface du patient par un procédé de triangulation en temps réel sur la base des valeurs de mesure détectée par la caméra (34, 36) durant un processus d'irradiation et à les comparer aux points de coordonnées de consigne afin de positionner le patient, dans lequel le dispositif d'analyse et de commande (38) comprend en plus un dispositif de mémorisation, dans lequel les points de coordonnées déterminés durant le processus d'irradiation sont enregistrés afin de documenter le processus d'irradiation, **caractérisé en ce qu'**au moins l'un des lasers spatiaux (18, 20, 22, 24) projette une ligne laser à la surface de l'appareil d'irradiation (16) dans la chambre d'irradiation, dans lequel au moins une caméra (34, 36) est conçue de manière à détecter la ligne laser projetée à la surface de l'appareil d'irradiation (16) par ledit laser spatial (18, 20, 22, 24) au moins, et dans lequel le dispositif d'analyse et de commande (38) est conçu de manière à déterminer les points de coordonnées le long de la ligne laser projetée à la surface de l'appareil d'irradiation (16) par un procédé de triangulation en temps réel sur la base des valeurs de mesure détectée par la caméra (34, 36) durant un processus d'irradiation, dans lequel le dispositif d'analyse et de commande (38) est encore conçu de manière à enregistrer les points de coordonnées déterminés durant le processus d'irradiation le long de la ligne laser projetée à la surface de l'appareil d'irradiation (16) dans le dispositif de mémorisation afin de documenter le processus d'irradiation.

2. Système selon la revendication 1, dans lequel les points de coordonnées de consigne ont été déterminés sur la base d'une scanographie du patient ayant précédé l'irradiation ou d'un autre procédé approprié d'imagerie de référence et sont stockés dans le dispositif de mémorisation du dispositif d'analyse et de commande.

3. Système selon la revendication 2, dans lequel les points de coordonnées de consigne ont été déterminés à partir des points de coordonnées d'intersection de la surface du patient déterminée dans le cadre de la scanographie ou d'un autre procédé approprié d'imagerie de référence avec au moins un plan traversant le centre d'une zone à irradier du patient, de préférence avec deux ou trois plans perpendiculaires entre eux et convergeant au centre d'une zone à irradier du patient.

4. Système selon l'une des revendications précédentes, dans lequel les points de coordonnées de consigne ont été déterminés, après que le patient a été positionné avant un processus d'irradiation à l'aide d'un procédé d'imagerie avec sa zone à irradier à l'isocentre de l'appareil d'irradiation, du fait que les points de coordonnées ont été déterminés le long de la ligne laser projetée à la surface du patient par un procédé de triangulation en temps réel au moyen du dispositif d'analyse et de commande (38) sur la base des valeurs de mesure détectée par la caméra (34, 36) et les points de coordonnées ainsi déterminés comme points de coordonnées de consigne sont stockés dans le dispositif de mémorisation du dispositif d'analyse et de commande.

5. Système selon l'une des revendications précédentes, comprenant encore un dispositif d'affichage, qui présente les points de coordonnées déterminés durant un processus d'irradiation et en option même les points de coordonnées de consigne en temps réel.

6. Système selon l'une des revendications précédentes, dans lequel le dispositif d'analyse et de commande (38) est encore conçu de manière à émettre un signal d'avertissement dans le cas d'un écart inadmissible entre les points de coordonnées déterminés et les points de coordonnées de consigne et / ou à procéder à une correction de la position du patient moyennant l'activation d'une commande de mouvement de la table de traitement (10).

7. Système selon l'une des revendications précédentes, dans lequel le dispositif d'analyse et de commande (38) est encore conçu de manière à détecter un mouvement respiratoire ou un autre mouvement du patient durant un processus d'irradiation.

8. Système selon la revendication 7, dans lequel dans lequel le dispositif d'analyse et de commande (38) est encore conçu de manière à activer l'appareil d'irradiation, de sorte qu'une irradiation n'est toujours réalisée que dans une position respiratoire prédéfinie ou dans une autre position de mouvement du patient.

9. Système selon l'une des revendications précédentes, dans lequel le dispositif d'analyse et de commande (38) est encore conçu de manière à déterminer les coordonnées de point de deux lignes laser convergeant au point d'intersection d'un faisceau central de l'appareil d'irradiation avec la surface du patient et à s'en servir pour définir la distance peau-point focal.

10. Système selon l'une des revendications précédentes, dans lequel au moins l'un des lasers spatiaux (18, 20, 22, 24) projette une ligne laser sur la surface de la table de traitement (10) à l'intérieur de la chambre d'irradiation, dans lequel au moins une caméra (34, 36) est conçue de manière à détecter la ligne laser projetée à la surface de la table de traitement (10) par ledit laser spatial (18, 20, 22, 24) au moins, et dans lequel le dispositif d'analyse et de commande (38) est conçu de manière à déterminer les points de coordonnées le long de la ligne laser projetée à la surface de la table de traitement (10) par un procédé de triangulation en temps réel sur la base des valeurs de mesure détectée par la caméra (34, 36) durant un processus d'irradiation.

11. Système selon la revendication 10, dans lequel le dispositif d'analyse et de commande (38) est encore conçu de manière à comparer les points de coordonnées déterminés le long de la ligne laser projetée sur la surface de la table de traitement (10) et / ou de l'appareil d'irradiation (16) aux points de coordonnées de consigne et à émettre un signal d'avertissement, notamment un signal d'avertissement de collision, dans le cas d'un écart inadmissible entre les points de coordonnées déterminés et les points de coordonnées de consigne.

12. Système selon l'une des revendications 10 ou 11, dans lequel le dispositif d'analyse et de commande (38) est encore conçu de manière à enregistrer les points de coordonnées déterminés durant le processus d'irradiation le long de la ligne laser projetée à la surface de la table de traitement (10) dans le dispositif de mémorisation afin de documenter le processus d'irradiation.

13. Système selon l'une des revendications précédentes, dans lequel au moins quatre lasers spatiaux (18, 20, 22, 24) sont prévus, dans lequel deux desdits quatre lasers spatiaux (18, 20, 22, 24) au moins sont disposés sur les côtés opposés de la table de traitement (10) et projettent respectivement une ligne laser horizontale latérale et une ligne transversale sur un patient reposant sur la table de traitement (10), dans lequel encore deux au moins desdits quatre lasers spatiaux (18, 20, 22, 24) au moins sont disposés au-dessus de la table de traitement (10), dont l'un au moins projette une ligne transversale sur un patient reposant sur la table de traitement (10), et dont l'autre projette une ligne longitudinale sur un patient reposant sur la table de traitement (10).

14. Système selon la revendication 13, dans lequel les lasers spatiaux latéraux (18, 20) comprennent respectivement deux sources laser, dont l'une projette la ligne laser horizontale latérale et l'autre la ligne transversale sur le patient reposant sur la table de traitement (10).

15. Système selon l'une des revendications 13 ou 14, dans lequel au moins deux caméras (34, 36) sont prévues, qui sont respectivement conçues pour détecter les lignes laser projetées par au moins deux lasers spatiaux latéraux (18, 20).
